# EUROPEAN PATENT APPLICATION

(11) **EP 4 834 935 A1**
(43) Date of publication of application: **04.11.2026**
(21) Application number: 24911247.5
(22) Date of filing: 25.12.2024
(51) Int. Cl.: C07K 16/28, A61K 31/337, A61K 31/282, C07K 16/22, A61P 35/00, A61K 39/395

(54) **USE OF ANTI-PD-1 ANTIBODY COMBINED WITH CHEMOTHERAPEUTIC AGENT IN TREATMENT OF TUMORS**

(30) Priority: 26.12.2023 CN 202311812121; 26.12.2023 CN 202311811929
(71) Applicant: Bio-Thera Solutions, Ltd., Guangzhou, Guangdong 510005 (CN)
(72) Inventor: WANG, Hao, Guangzhou, Guangdong 510530 (CN); SONG, Shuqiang, Guangzhou, Guangdong 510530 (CN); YUE, Haitao, Guangzhou, Guangdong 510530 (CN); YU, Jin-Chen, Guangzhou, Guangdong 510530 (CN)
(74) Representative: Engelhard, Markus
(86) International application number: PCT/CN2024/142361
(87) International publication number: WO 2025/140324

(57) **Abstract**

Provided is a use of an anti-PD-1 antibody combined with a chemotherapeutic agent in the treatment of tumors such as cervical cancer or endometrial cancer. In some embodiments, the chemotherapeutic agent comprises a taxane drug and/or a platinum drug. In some embodiments, the use further comprises a use of the anti-PD-1 antibody combined with bevacizumab.

## Description

### TECHNICAL FIELD

The present disclosure pertains to the field of bio-pharmaceuticals, and particularly relates to use of an anti-PD-1 antibody in combination with a chemotherapeutic agent in treating a tumor, such as cervical cancer or endometrial cancer.

### BACKGROUND

Programmed cell death receptor-1 (PD-1) is an immunoinhibitory receptor expressed on activated T cells, B cells, and myeloid cells, and is a member of the CD28 immunoglobulin superfamily. PD-1 is a 55 kDa type I transmembrane glycoprotein comprising an Ig variable domain for binding to a ligand, and a cytoplasmic tail responsible for binding to a signaling molecule. The PD-1 cytoplasmic tail contains two tyrosine-based signaling motifs, an immunoreceptor tyrosine-based inhibitory motif (ITIM) and an immunoreceptor tyrosine-based switch motif (ITSM).

The incidence of cervical cancer ranks first among malignant tumors of the female reproductive system. According to the data from the World Health Organization (WHO), there are 530,000 new cases worldwide each year, and about 250,000 women die from cervical cancer, with the deaths of women in developing countries accounting for 80% of the deaths of women worldwide due to cervical cancer. There is an unmet clinical need. Therefore, new therapeutic methods are required to improve the prognosis of these patients.

Endometrial cancer is one of the common gynecological malignant tumors, and its incidence is increasing year by year. Currently, there is still a great clinical need for the first-line treatment of endometrial cancer.

### SUMMARY

The present disclosure provides a method or use of an anti-PD-1 antibody in combination with a chemotherapeutic agent for treating a tumor. In some embodiments, the present disclosure provides use of an anti-PD-1 antibody in combination with a chemotherapeutic agent in the preparation of a pharmaceutical composition for treating a tumor. In some embodiments, the present disclosure provides use of an anti-PD-1 antibody in the preparation of a medicament for treating a tumor in combination with a chemotherapeutic agent. In some embodiments, the present disclosure provides use of an anti-PD-1 antibody in treating a tumor in combination with a chemotherapeutic agent. In some embodiments, the present disclosure provides a method for treating a patient with a tumor, comprising administering to the patient a therapeutically effective amount of an anti-PD-1 antibody and a chemotherapeutic agent. In some embodiments, the tumor is cervical cancer or endometrial cancer.

In some embodiments, the anti-PD-1 antibody comprises HCDR1 set forth in SEQ ID NO: 1, HCDR2 set forth in SEQ ID NO: 2, HCDR3 set forth in SEQ ID NO: 3, LCDR1 set forth in SEQ ID NO: 4, LCDR2 set forth in SEQ ID NO: 5, and LCDR3 set forth in SEQ ID NO: 6.

In some embodiments, a heavy chain variable region of the anti-PD-1 antibody comprises the sequence set forth in SEQ ID NO: 7, a sequence having at least 80% identity to the sequence set forth in SEQ ID NO: 7, or an amino acid sequence having one or more conservative amino acid substitutions as compared to the sequence set forth in SEQ ID NO: 7; and/or
a light chain variable region of the anti-PD-1 antibody comprises the sequence set forth in SEQ ID NO: 8, a sequence having at least 80% identity to the sequence set forth in SEQ ID NO: 8, or an amino acid sequence having one or more conservative amino acid substitutions as compared to the sequence set forth in SEQ ID NO: 8.

In some embodiments, the heavy chain variable region of the anti-PD-1 antibody comprises the sequence set forth in SEQ ID NO: 7, and the light chain variable region of the anti-PD-1 antibody comprises the sequence set forth in SEQ ID NO: 8.

In some embodiments, a heavy chain of the anti-PD-1 antibody comprises the sequence set forth in SEQ ID NO: 9, a sequence having at least 80% identity to the sequence set forth in SEQ ID NO: 9, or an amino acid sequence having one or more conservative amino acid substitutions as compared to the sequence set forth in SEQ ID NO: 9; and/or
a light chain of the anti-PD-1 antibody comprises the sequence set forth in SEQ ID NO: 10, a sequence having at least 80% identity to the sequence set forth in SEQ ID NO: 10, or an amino acid sequence having one or more conservative amino acid substitutions as compared to the sequence set forth in SEQ ID NO: 10.

In some embodiments, the anti-PD-1 antibody is antibody A, a heavy chain of antibody A comprises the sequence set forth in SEQ ID NO: 9, and a light chain of antibody A comprises the sequence set forth in SEQ ID NO: 10; antibody A comprises two heavy chains with identical sequences and two light chains with identical sequences.

In some embodiments, the anti-PD-1 antibody at a dose of about 50-600 mg or a formulation containing the anti-PD-1 antibody at this dose is administered. In some embodiments, the anti-PD-1 antibody at a dose of about 200-400 mg or a formulation containing the anti-PD-1 antibody at this dose is administered. In some embodiments, the anti-PD-1 antibody at a dose of about 50 mg, about 60 mg, about 80 mg, about 120 mg, about 200 mg, about 250 mg, about 290 mg, about 300 mg, about 330 mg, about 380 mg, about 400 mg, about 434 mg, about 480 mg, about 500 mg, about 567 mg, about 580 mg, about 600 mg, or a range between any two of these values (inclusive) or any value therein, or a formulation containing the anti-PD-1 antibody at this dose is administered.

In some embodiments, the administration is performed about once every week, or about once every 2 weeks, about once every 3 weeks, about once every 4 weeks, about once every 5 weeks, about once every 6 weeks, or about once every 7 weeks. In some embodiments, the administration is performed once every 2 weeks, every 3 weeks, or every 4 weeks. In some embodiments, the administration is performed once every 3 weeks.

In some embodiments, one treatment cycle is at least 1 week, at least 2 weeks, at least 3 weeks, at least 4 weeks, at least 5 weeks, at least 6 weeks, or at least 7 weeks. In some embodiments, one treatment cycle is 1 week, 2 weeks, 3 weeks, 4 weeks, 5 weeks, 6 weeks, 7 weeks, or a range between any two of these values (inclusive) or any value therein.

In some embodiments, the anti-PD-1 antibody is administered at a dose of about 200-400 mg once every 2-4 weeks. In some embodiments, the anti-PD-1 antibody is administered at a dose of about 100 mg, 300 mg, or 600 mg once every 3 weeks. In some embodiments, the anti-PD-1 antibody is administered at a dose of about 200-220 mg once every 2 weeks. In some embodiments, the anti-PD-1 antibody is administered at a dose of about 300 mg once every 3 weeks. In some embodiments, the anti-PD-1 antibody is administered at a dose of about 380-400 mg once every 4 weeks.

In some embodiments, the present disclosure provides a method or use of an anti-PD-1 antibody in combination with a chemotherapeutic agent for treating a patient with cervical cancer.

In some embodiments, the present disclosure discloses a method or use of an anti-PD-1 antibody in combination with a chemotherapeutic agent for treating a patient with endometrial cancer.

In some embodiments, the chemotherapeutic agent is a taxane, a platinum-based drug, or a combination thereof. In some embodiments, the chemotherapeutic agent is a combination of a taxane and a platinum-based drug.

In some embodiments, the taxane is paclitaxel or paclitaxel liposome.

In some embodiments, the platinum-based drug is cisplatin or carboplatin.

In some embodiments, the chemotherapeutic agent is selected from the following groups: paclitaxel and cisplatin, paclitaxel and carboplatin, paclitaxel liposome and cisplatin, and paclitaxel liposome and carboplatin.

In some embodiments, the paclitaxel is administered at a dose of about 50-300 mg/m² based on body surface area. In some embodiments, the paclitaxel is administered at a dose of about 135-175 mg/m² based on body surface area. In some embodiments, the paclitaxel is administered at a dose of about 100, 135, 150, 175, 200, 250, or 300 mg/m² based on body surface area, or a range between any two of these values (inclusive) or any value therein. In some embodiments, the paclitaxel is administered once every week, every 2 weeks, every 3 weeks, or every 4 weeks. In some embodiments, the paclitaxel is administered once every 3 weeks. In some embodiments, the paclitaxel is administered at a dose of about 175 mg/m² based on body surface area once every 3 weeks.

In some embodiments, the cisplatin is administered at a dose of about 10-100 mg/m² based on body surface area. In some embodiments, the cisplatin is administered at a dose of about 10, 20, 30, 40, 50, 60, 70, 80, 90, or 100 mg/m² based on body surface area, or a range between any two of these values (inclusive) or any value therein. In some embodiments, the cisplatin is administered once every week, every 2 weeks, every 3 weeks, or every 4 weeks. In some embodiments, the cisplatin is administered once every 3 weeks. In some embodiments, the cisplatin is administered at a dose of about 50 mg/m² based on body surface area once every 3 weeks.

In some embodiments, the carboplatin is administered at a dose of AUC 4-7 mg/mL/min. In some embodiments, the carboplatin is administered at a dose achieving an AUC of about 4, 5, 6, or 7 mg/mL/min. In some embodiments, the carboplatin is administered once every week, every 2 weeks, every 3 weeks, or every 4 weeks. In some embodiments, the carboplatin is administered once every 3 weeks. In some embodiments, the carboplatin is administered at a dose of AUC 5 mg/mL/min once every 3 weeks.

In some embodiments, the present disclosure provides a method for treating a patient with cervical cancer, comprising administering to the patient a therapeutically effective amount of an anti-PD-1 antibody (e.g., antibody A) and a chemotherapeutic agent, wherein the chemotherapeutic agent is selected from the following groups: paclitaxel and cisplatin, paclitaxel and carboplatin, paclitaxel liposome and cisplatin, and paclitaxel liposome and carboplatin. In some embodiments, the present disclosure provides use of an anti-PD-1 antibody (e.g., antibody A), paclitaxel/paclitaxel liposome, and cisplatin/carboplatin in the preparation of a pharmaceutical composition for treating cervical cancer. In some embodiments, the present disclosure provides use of an anti-PD-1 antibody (e.g., antibody A) in the preparation of a medicament for treating cervical cancer in combination with paclitaxel/paclitaxel liposome and cisplatin/carboplatin. In some embodiments, the present disclosure provides use of an anti-PD-1 antibody (e.g., antibody A) in combination with paclitaxel/paclitaxel liposome and cisplatin/carboplatin in treating cervical cancer.

In some embodiments, the present disclosure provides a method or use of an anti-PD-1 antibody in combination with paclitaxel/paclitaxel liposome and cisplatin/carboplatin for treating a patient with cervical cancer, the method comprising administering as follows: (1) administering to the patient the anti-PD-1 antibody (e.g., antibody A) at a dose of about 300 mg once every 3 weeks, (2) administering the paclitaxel or paclitaxel liposome once every 3 weeks, and (3) administering the cisplatin or carboplatin once every 3 weeks.

In some embodiments, the method or use further comprises administering bevacizumab. In some embodiments, the bevacizumab comprises Avastin or a biosimilar thereof, e.g., Pobevcy, Pusintin, MIL60, Hanbeitai, Airuituo, Boyounuo, Byvasda, Ankeda, Alymsys, Zirabev, Mvasi, Vegzelma, Onbevzi, Oyavas, Aybintio, or Abevmy. In some embodiments, the bevacizumab is administered at a dose of about 15 mg/kg once every 3 weeks.

In some embodiments, the method or use comprises administering to the patient a therapeutically effective amount of an anti-PD-1 antibody (e.g., antibody A), a chemotherapeutic agent, and bevacizumab, wherein the chemotherapeutic agent is selected from the following groups: paclitaxel and cisplatin, paclitaxel and carboplatin, paclitaxel liposome and cisplatin, and paclitaxel liposome and carboplatin.

In some embodiments, the present disclosure provides a method for treating a patient with cervical cancer, comprising: (1) administering to the patient an anti-PD-1 antibody (e.g., antibody A) at a dose of about 300 mg once every 3 weeks, (2) administering paclitaxel or paclitaxel liposome once every 3 weeks, (3) administering cisplatin or carboplatin once every 3 weeks, and (4) administering bevacizumab at a dose of about 15 mg/kg once every 3 weeks.

In some embodiments, the paclitaxel is administered at a dose of about 175 mg/m² based on body surface area once every 3 weeks.

In some embodiments, the cisplatin is administered at a dose of about 50 mg/m² based on body surface area once every 3 weeks.

In some embodiments, the carboplatin is administered at a dose of AUC 5 mg/mL/min once every 3 weeks.

In some embodiments, compared to patients undergoing chemotherapy, the administration of the anti-PD-1 antibody in combination with the chemotherapeutic agent prolongs the progression-free survival (PFS) and overall survival (OS) of the patient. In some embodiments, compared to patients undergoing chemotherapy, the administration of the anti-PD-1 antibody in combination with the chemotherapeutic agent results in an improvement in at least one selected from the following: objective response rate (ORR), disease control rate (DCR), duration of response (DOR), time to progression (TTP), progression-free survival (PFS), and overall survival (OS). In some embodiments, the Response Evaluation Criteria in Solid Tumors is RECIST 1.1. In some embodiments, the Response Evaluation Criteria in Solid Tumors is iRECIST.

In some embodiments, the present disclosure provides a method or use of an anti-PD-1 antibody in combination with a chemotherapeutic agent for first-line treatment of a patient with cervical cancer.

In some embodiments, the cervical cancer comprises squamous cell carcinoma, adenocarcinoma, adenosquamous carcinoma, and clear cell carcinoma. In some embodiments, the cervical cancer is squamous cell carcinoma. In some embodiments, the cervical cancer is adenocarcinoma. In some embodiments, the cervical cancer is adenosquamous carcinoma. In some embodiments, the cervical cancer is clear cell carcinoma.

In some embodiments, the cervical cancer comprises metastatic cervical cancer, recurrent cervical cancer, and persistent cervical cancer.

In some embodiments, the cervical cancer is PD-L1-positive (CPS ≥ 1) cervical cancer. In some embodiments, the cervical cancer is PD-L1-positive (CPS ≥ 1) metastatic cervical cancer, recurrent cervical cancer, or persistent cervical cancer.

In some embodiments, the patient with cervical cancer is not amenable to radical surgery and/or radical radiotherapy or chemoradiotherapy.

In some embodiments, the patient with cervical cancer has not received any systemic anti-tumor treatment for recurrent or metastatic cervical cancer.

In some embodiments, the patient with cervical cancer has not previously received any treatment targeting tumor immune mechanisms of action, such as anti-angiogenic therapy (e.g., bevacizumab), treatment with an immune checkpoint inhibitor (e.g., an anti-PD-1 antibody, an anti-PD-L1 antibody, or an anti-CTLA-4 antibody), or treatment targeting immune co-stimulatory factors (e.g., antibodies directed against ICOS, CD40, CD137, GITR, or OX40 target).

In some embodiments, the patient with cervical cancer has previously received multiple cycles of treatment with paclitaxel-based drugs (including paclitaxel for injection) and platinum-based drugs (including cisplatin and carboplatin) during the adjuvant or neoadjuvant phase or the radical chemoradiotherapy phase.

In some embodiments, the present disclosure provides a method for treating a patient with endometrial cancer, comprising administering to the patient a therapeutically effective amount of an anti-PD-1 antibody (e.g., antibody A), paclitaxel, and carboplatin. In some embodiments, the present disclosure provides use of an anti-PD-1 antibody (e.g., antibody A), paclitaxel, and carboplatin in the preparation of a pharmaceutical composition for treating endometrial cancer. In some embodiments, the present disclosure provides use of an anti-PD-1 antibody (e.g., antibody A) in the preparation of a medicament for treating endometrial cancer in combination with paclitaxel and carboplatin. In some embodiments, the present disclosure provides use of an anti-PD-1 antibody (e.g., antibody A) in combination with paclitaxel and carboplatin in treating endometrial cancer.

In some embodiments, the present disclosure provides a method or use of an anti-PD-1 antibody in combination with paclitaxel and carboplatin for treating a patient with endometrial cancer, the method comprising: (1) administering to the patient the anti-PD-1 antibody (e.g., antibody A) at a dose of about 300 mg once every 3 weeks, (2) administering the paclitaxel once every 3 weeks, and (3) administering the carboplatin once every 3 weeks.

In some embodiments, the paclitaxel is administered at a dose of about 175 mg/m² based on body surface area once every 3 weeks.

In some embodiments, the carboplatin is administered at a dose of AUC 5 mg/mL/min once every 3 weeks.

In some embodiments, the present disclosure provides a method for treating a patient with endometrial cancer, comprising: (1) administering to the patient an anti-PD-1 antibody (e.g., antibody A) at a dose of about 300 mg once every 3 weeks, (2) administering to the patient in need thereof paclitaxel at a dose of about 175 mg/m² based on body surface area once every 3 weeks, and (3) administering carboplatin at a dose of AUC 5 mg/mL/min once every 3 weeks.

In some embodiments, the present disclosure provides a method or use of an anti-PD-1 antibody in combination with a chemotherapeutic agent for first-line treatment of a patient with endometrial cancer.

In some embodiments, the endometrial cancer is recurrent or metastatic endometrial cancer. In some embodiments, the endometrial cancer is one of the following: a) a newly diagnosed stage III disease (a lesion evaluated as measurable according to RECIST 1.1 after surgery or diagnostic biopsy), b) a newly diagnosed stage IV disease (with or without a lesion after surgery or diagnostic biopsy), and c) a recurrent disease (a lesion evaluated as measurable or non-measurable according to RECIST 1.1), with little likelihood of cure by surgery alone or in combination. In some embodiments, the recurrent disease has been treated with prior chemotherapy in an adjuvant setting (as part of early/adjuvant anti-cancer treatment).

In some embodiments, the endometrial cancer is mismatch repair protein-deficient (dMMR) endometrial cancer (Bonneville R, et al.; JCO Precis Oncol. 2017; 2017: PO.17.00073). In some embodiments, the endometrial cancer is dMMR recurrent or metastatic endometrial cancer. In some embodiments, the endometrial cancer is MSI-H/dMMR.

In some embodiments, the patient with endometrial cancer has not received first-line systemic anti-cancer treatment.

In some embodiments, the patient with endometrial cancer has not previously received any treatment targeting tumor immune mechanisms of action, such as treatment with an immune checkpoint inhibitor (e.g., an anti-PD-1 antibody, an anti-PD-L1 antibody, or an anti-CTLA-4 antibody) or treatment targeting immune co-stimulatory factors (e.g., antibodies directed against ICOS, CD40, CD137, GITR, or OX40 target).

In some embodiments, the anti-PD-1 antibody (or formulation), taxane (e.g., paclitaxel or paclitaxel liposome for injection), platinum-based drug (e.g., cisplatin or carboplatin), or bevacizumab is administered by intravenous infusion (i.v.). In some embodiments, the duration of the intravenous infusion of the anti-PD-1 antibody (or formulation) is about 30-90 minutes. In some embodiments, the duration of the intravenous infusion of the anti-PD-1 antibody (or formulation) is about 30-60 minutes. In some embodiments, bevacizumab (or an injection thereof) is administered by intravenous infusion, and the duration of the intravenous infusion is about 30-90 minutes. In some embodiments, the duration of the intravenous infusion of bevacizumab (or an injection thereof) is about 30, 60, or 90 minutes.

In some embodiments, the patient receives treatment with an anti-PD-1 antibody (e.g., antibody A) and a chemotherapeutic agent for one treatment cycle. In some embodiments, the patient receives treatment with an anti-PD-1 antibody (e.g., antibody A), a chemotherapeutic agent, and bevacizumab for one treatment cycle. In some embodiments, the patient receives treatment for multiple (e.g., 2-35, e.g., 5, 10, 15, 20, 25, 30, 35, or a range between any two of these values (inclusive) or any value therein, with up to 6 cycles of chemotherapy) treatment cycles. In some embodiments, the patient receives treatment until the condition is alleviated and no treatment is required.

The "combination" described herein is a mode of administration, which includes various situations where two or more drugs are administered sequentially or simultaneously. In some embodiments, the anti-PD-1 antibody and the chemotherapeutic agent are each separate dosage units administered in combination. In some embodiments, the order of administration is: (1) anti-PD-1 antibody, (2) paclitaxel or paclitaxel liposome for injection, and (3) cisplatin or carboplatin. In some embodiments, the anti-PD-1 antibody, the chemotherapeutic agent, and bevacizumab are each separate dosage units administered in combination. In some embodiments, the order of administration is: (1) anti-PD-1 antibody, (2) paclitaxel or paclitaxel liposome for injection, (3) cisplatin or carboplatin, and (4) bevacizumab.

In another aspect, the present disclosure further provides a kit comprising an anti-PD-1 antibody (or formulation), a chemotherapeutic agent (or formulation), and instructions for directing administration of the anti-PD-1 antibody (or formulation) and the chemotherapeutic agent (or formulation) to a patient in need thereof. In some embodiments, the present disclosure further discloses a kit comprising a combination (or formulation) of an anti-PD-1 antibody and a chemotherapeutic agent, and an instruction for directing administration of the combination (or formulation) of the anti-PD-1 antibody and the chemotherapeutic agent to a patient in need thereof. In some embodiments, the kit further comprises an anti-VEGF antibody (or formulation), e.g., bevacizumab, and an instruction for directing administration of the anti-VEGF antibody (or formulation), e.g., bevacizumab, to a patient in need thereof.

In another aspect, the present disclosure further provides a pharmaceutical composition, which comprises an anti-PD-1 antibody and a chemotherapeutic agent and is suitable for injection, such as a pharmaceutical composition for bolus injection or a pharmaceutical composition for infusion (drip). In some embodiments, the pharmaceutical composition further comprises an anti-VEGF antibody (or formulation), e.g., bevacizumab. In some embodiments, the pharmaceutical composition comprises at least 0.1% of the anti-PD-1 antibody and at least 0.1% of the chemotherapeutic agent. The percentage of the antibody and the chemotherapeutic agent may vary and is between about 2% and about 90% by weight of a given dosage form. The amount of the anti-PD-1 antibody, the anti-VEGF antibody, and the chemotherapeutic agent in such a therapeutically useful pharmaceutical composition may be an effective amount for administration.

In another aspect, the present disclosure further provides a preparation method for the above pharmaceutical composition, which comprises mixing the anti-PD-1 antibody and the chemotherapeutic agent (or a combination of the anti-PD-1 antibody and the chemotherapeutic agent) described herein with a pharmaceutically acceptable carrier suitable for injection (e.g., water for injection and normal saline). The method for mixing the anti-PD-1 antibody and the chemotherapeutic agent described above with the pharmaceutically acceptable carrier is generally known in the art.

In some embodiments, the administration of the anti-PD-1 antibody in combination with the chemotherapeutic agent prolongs the progression-free survival (PFS) and overall survival (OS) of the patient. In some embodiments, the administration of the anti-PD-1 antibody in combination with the chemotherapeutic agent results in an improvement in at least one selected from the following: objective response rate (ORR), disease control rate (DCR), duration of response (DOR), time to progression (TTP), progression-free survival (PFS), and overall survival (OS).

In some embodiments, the combination regimen of the present disclosure has excellent therapeutic effects.

### Terms

Unless otherwise stated, each of the following terms shall have the meaning described below.

### Definitions

It should be noted that the term "an" entity refers to one or more of the entities. For example, "an antibody" should be interpreted as one or more antibodies. As such, the terms "a" (or "an"), "one or more", and "at least one" are used interchangeably herein.

As used herein, the term "contain", "comprise", or "include" means that the compositions, methods, and the like include the recited elements, such as components or steps, and do not exclude the others. "Consisting essentially of..." means that the compositions and methods exclude other elements that have a fundamental impact on the characteristics of the combination, but do not exclude elements that do not substantially affect the characteristics of the compositions or methods. "Consisting of..." means that elements not specifically recited are excluded.

"Conservative amino acid substitution" refers to the substitution of one amino acid residue with another amino acid residue having a side chain (R group) of similar chemical properties (e.g., charge or hydrophobicity). Generally, conservative amino acid substitutions do not substantially alter the functional properties of the protein. Examples of amino acid classes having a side chain of similar chemical properties include: 1) an aliphatic side chain: glycine, alanine, valine, leucine, and isoleucine; 2) an aliphatic hydroxyl side chain: serine and threonine; 3) an amide-containing side chain: asparagine and glutamine; 4) an aromatic side chain: phenylalanine, tyrosine, and tryptophan; 5) a basic side chain: lysine, arginine, and histidine; and 6) an acidic side chain: aspartic acid and glutamic acid.

The number of amino acids undergoing "conservative amino acid substitutions in VL or VH" may be about 1, about 2, about 3, about 4, about 5, about 6, about 8, about 9, about 10, about 11, about 13, about 14, about 15, or a range between any two of these values (inclusive) or any value therein. The number of amino acids undergoing "conservative amino acid substitutions in a heavy chain or a light chain" may be about 1, about 2, about 3, about 4, about 5, about 6, about 8, about 9, about 10, about 11, about 13, about 14, about 15, about 18, about 19, about 22, about 24, about 25, about 29, about 31, about 35, about 38, about 41, about 45, or a range between any two of these values (inclusive) or any value therein.

"Homology", "identity", or "similarity" refers to sequence similarity between two peptides or between two nucleic acid molecules. Homology can be determined by comparing the positions that can be aligned in the sequences. When a position of the compared sequences is occupied by the same base or amino acid, the molecules are homologous at that position. The degree of homology between the sequences is a function of the number of matching or homologous positions shared by the sequences.

"At least 80% identity" refers to about 80% identity, about 81% identity, about 82% identity, about 83% identity, about 85% identity, about 86% identity, about 87% identity, about 88% identity, about 90% identity, about 91% identity, about 92% identity, about 94% identity, about 95% identity, about 98% identity, about 99% identity, or a range between any two of these values (inclusive) or any value therein.

"Antibody" or "antigen-binding fragment" refers to a polypeptide or polypeptide complex that specifically recognizes and binds to an antigen. The antibody may be an intact antibody and any antigen-binding fragment thereof or a single chain thereof. The term "antibody" thus includes any protein or peptide comprising at least a portion of an immunoglobulin molecule that has biological activity for binding to an antigen. The antibody and the antigen-binding fragment include, but are not limited to, complementarity determining regions (CDRs) of a heavy or light chain or a ligand-binding portion thereof, heavy chain variable regions (VHs), light chain variable regions (VLs), heavy chain constant regions (CHs), light chain constant regions (CLs), framework regions (FRs), or any portion thereof, or at least a portion of a binding protein. CDRs include light chain CDRs (LCDR1-3) and heavy chain CDRs (HCDR1-3).

The term "antibody" includes a wide variety of polypeptides that can be biochemically distinguished. It will be appreciated by those skilled in the art that the classes of heavy chains include gamma, mu, alpha, delta, or epsilon (γ, µ, α, δ, or ε), and some subclasses (e.g., γ1-γ4). The nature of this chain determines that the "type" of the antibody is IgG, IgM, IgA, IgD, or IgE. Immunoglobulin subclasses (isotypes), such as IgG1, IgG2, IgG3, and IgG4, have been well characterized, and the functional specificity imparted is also known. All types of immunoglobulins are within the protection scope of the present disclosure. In some embodiments, the immunoglobulin molecule is of the IgG type. These four chains are connected in a "Y" configuration through disulfide bonds, wherein the light chain starts at the opening of the "Y" configuration and extends through the variable region to surround the heavy chain.

The antibodies, antigen-binding fragments, or derivatives described herein include, but are not limited to, polyclonal antibodies, monoclonal antibodies, multispecific antibodies, fully human antibodies, humanized antibodies, primatized antibodies, chimeric antibodies, singlechain variable fragments (scFvs), and epitope-binding fragments (e.g., Fab, Fab', and F(ab')₂).

Light chains can be classified into kappa (κ) or lambda (λ). Each heavy chain may be connected to a κ or λ light chain. In general, when an immunoglobulin is produced by a hybridoma, a B cell, or a genetically engineered host cell, the light and heavy chains are connected by covalent bonds, and the "tail" portions of the two heavy chains are connected by covalent disulfide bonds or non-covalent bonds. In the heavy chains, the amino acid sequence extends from the N-terminus at the forked end of the Y configuration to the C-terminus at the bottom of each chain. The immunoglobulin κ light chain variable region is Vκ; and the immunoglobulin λ light chain variable region is V_{λ}.

The light chain variable region (VL) and the heavy chain variable region (VH) determine the antigen recognition and specificity. The light chain constant region (CL) and the heavy chain constant region (CH) impart important biological properties, such as secretion, transplacental movement, Fc receptor binding, and complement fixation. By convention, the numbering of amino acids in the constant regions increases as they become further away from the antigen-binding site of the antibody or amino terminus. The N-terminal portion is the variable region, and the C-terminal portion is the constant region; the CH3 and CL domains actually comprise the carboxyl termini of the heavy chain and light chain, respectively.

Where the terms used and/or accepted in the art have two or more definitions, the definitions of the terms used herein include all of these meanings unless explicitly indicated as opposite. One specific example is the use of the term "complementarity determining regions" ("CDRs") to describe non-contiguous antigen-binding sites found within the variable regions of heavy and light chain polypeptides.

CDRs defined according to Kabat and Chothia include overlaps or subsets of amino acid residues when compared to each other. Nevertheless, it is within the scope of the present disclosure to apply any definition to refer to the CDRs of an antibody or a variant thereof. The exact number of residues comprising a specific CDR will vary according to the sequence and size of the CDR. Those skilled in the art can generally determine which specific residues a CDR comprises based on the variable region amino acid sequence of an antibody.

Kabat et al. also defined a numbering system applicable to the variable region sequence of any antibody. Those of ordinary skill in the art can apply the "Kabat numbering" system to any variable region sequence without depending on other experimental data beyond the sequence itself. "Kabat numbering" refers to the numbering system proposed by Kabat et al., U.S. Dept. of Health and Human Services in Sequence of Proteins of Immunological Interest (1983). The EU or Chothia numbering system can also be applicable to the antibody.

"Treatment" refers to both therapeutic treatment and prophylactic or preventative measures, the purpose of which is to prevent, slow down, ameliorate, or halt undesirable physiological changes or disorders, such as the progression of a disease, including, but not limited to, alleviation of symptoms, diminishment of extent of disease, stabilization (i.e., not worsening) of disease state, delay or slowing of disease progression, amelioration, palliation, alleviation, or elimination (whether partial or total) of disease state, prolongation of life expectancy in the absence of treatment, and the like, as either detectable or undetectable. Patients in need of treatment include patients already with a condition or disorder, patients susceptible to a condition or disorder, or patients in need of prevention of the condition or disorder, or patients who may or are expected to benefit from administration of the antibody or the composition described herein for detection, diagnostic procedures, and/or treatment.

"About" refers to a general error range for corresponding values as readily understood by those skilled in the relevant art. In some embodiments, the term "about" mentioned herein refers to the values described and a range of ±10%, ±5%, or ±1% thereof.

"Effective amount" refers to the amount of an active compound or medicament that results in a biological or medical response in a tissue, system, animal, individual, or human; the effective amount is sought by researchers, vets, doctors, or other clinical doctors.

PK parameters of single administration: Cₘₐₓ, Tₘₐₓ, T_{1/2}, CL, Vd, Ke, MRT, AUC_{(0-τ)}, and AUC_{(0-∞)}.

| | |
|---|---|
| Cₘₐₓ | Peak concentration. Directly obtained from the measured plasma concentration-time data. |
| Tₘₐₓ | Time to maximum plasma concentration. Directly obtained from the measured plasma concentration-time data. |
| t_{1/2} | Terminal elimination half-life. t_{1/2} = ln2/λ_{z} |
| CL | Apparent clearance. CL = Dₚₒ/AUC_{0-∞} |
| Vd | Apparent volume of distribution. Vd = Dₚₒ/AUC_{0-∞}/λ_{z} |
| Ke (λ_{z}) | Elimination rate constant, a slope of the terminal segment of a semilogarithmic drug concentration-time curve calculated by using the linear regression method. |
| MRT₀₋ₜ | Mean residence time, calculated by the formula: AUMC₀₋ₜ/AUC₀₋ₜ, where the calculation formula for AUMC₀₋ₜ is: AUMC_{(i, i+1)} = ()()/2, and AUMC is the sum of all AUMC_{(i, i+1)} |
| MRT_{0-∞} | Calculation formula: AUMC_{0-∞}/AUC_{0-∞}, where AUMC_{0-∞} = AUMC₀₋ₜ + Tₗₐₛₜ × \|Cₗₐₛₜ\|/λz + \|Cₗₐₛₜ\|/(λz × λz) |
| AUC₀₋ₜ | Area under the curve from zero to the time at which the lowest plasma concentration can be detected. Calculated by the linear trapezoidal rule: AUC_{(i, i+1)} = (Tᵢ₊₁ - Tᵢ)(Cᵢ + Cᵢ₊₁)/2, where AUC₀₋ₜ is the sum of all AUC_{(i,} i+1). |
| 58AUC_{0-∞} | Area under the curve of time extrapolated from zero to infinity. AUC_{0-∞} = AUC₀₋ₜ + Cₜ/λ_{z} (Ct is the last measurable plasma concentration). |
| AUC__{%Extrap} | The proportion of AUC theoretically extrapolated from the last point to infinity relative to AUC_{0-∞} |

PK parameters of multiple administrations: C_{max, ss}, C_{avg, ss}, C_{min, ss}, AUC_{(0-τ)ss}, AUC_{(0-∞)ss}, Tₘₐₓ, ₛₛ, T_{1/2, ss}, CL, Vₛₛ, Ke, MRT, accumulation index (R_{ac}), and fluctuation index DF.

| | |
|---|---|
| C_{max, ss} | Peak concentration at steady state. Directly obtained from the measured plasma concentration-time data at steady state within administration intervals. |
| C_{min, ss} | Minimum concentration at steady state. Directly obtained from the measured plasma concentration-time data at steady state within administration intervals. |
| C_{avg, ss} | Mean plasma concentration at steady state within administration intervals. Cₐᵥ = AUC_{0-τ}/τ, where τ is the administration interval |
| AUC₍₀₋ₜ₎ₛₛ | Area under the curve at steady state from zero to the time at which the lowest plasma concentration can be detected. Calculated by the linear trapezoidal rule: AUC_{(i, i+1), ss} = (Tᵢ₊₁ - Tᵢ)(Cᵢ + Cᵢ₊₁)/2, where AUC_{0-t, ss} is the sum of all AUC_{(i, i+1), ss}. |
| AUC_{(0-∞)ss} | Area under the curve of time at steady state extrapolated from zero to infinity. |
| | AUC_{0-∞, ss} = AUC_{0-t, ss} + Cₜ/λ_{z} (Cₜ is the last measurable plasma concentration at steady state). |
| T_{max, ss} | Time to maximum plasma concentration. Directly obtained from the measured plasma concentration-time data. |
| t_{1/2, ss} | Terminal elimination half-life. t_{1/2, ss} = ln2/λ_{z} |
| CLₛₛ | Apparent clearance at steady state. CLₛₛ = Dₚₒ/AUCₛₛ. |
| Vd | Volume of distribution at steady state, Vd = Dₚₒ/AUC_{0-∞}, ₛₛ/λ_{z} |
| Ke (λ_{z}) | Elimination rate constant, a slope of the terminal segment of a semilogarithmic drug concentration-time curve calculated by using the linear regression method. |
| MRT_{0-∞, ss} | Calculation formula: AUMC_{0-∞, ss}/AUC_{0-∞, ss}, where AUMC_{0-∞, ss} = AUMC_{0-t, ss} + Tₗₐₛₜ × \|Cₗₐₛₜ\|/λ_{z} ⁺ \|Cₗₐₛₜ\|/(λ_{z} × λ_{z}) |
| Rac | Accumulation ratio. Rac = |
| DF | The degree of fluctuation. DF = (C_{max, ss} - C_{min, ss})/C_{avg, ss} |
| AUC__{%Extrap} | The proportion of AUC theoretically extrapolated from the last point to infinity relative to AUC_{0-∞} |

The U.S. Eastern Cooperative Oncology Group (ECOG) formulated a simplified performance status scale. Patients are classified into 6 levels of 0-5 for performance status, with the ECOG performance status scoring system assigning scores of 0, 1, 2, 3, 4, and 5.

| **Grade** | **U.S. Eastern Cooperative Oncology Group performance status** |
|---|---|
| 0 | Fully normal motility, without difference from the pre-disease motility |
| 1 | Able to walk freely and engage in light physical activities, including general housework or office work, but unable to engage in strenuous physical activities |
| 2 | Able to walk freely and carry on self-care, but unable to work; able to get up and move for no less than half of the day |
| 3 | Capable of only limited self-care; confined to bed or wheelchair more than half of the day |
| 4 | Totally confined to bed; completely unable to carry on self-care; totally confined to bed or wheelchair. |
| 5 | Dead |

Tumor assessment is performed according to the Response Evaluation Criteria in Solid Tumors (RECIST 1.1) (Eisenhauer, E A et al., Eur J Cancer. 2009; 45(2):228-247) and iRECIST (Seymour L, et al., Lancet Oncol. 2017; 18:e143-e152), with RECIST 1.1 as the primary evaluation criterion.

Objective response rate (ORR), duration of response (DOR), disease control rate (DCR), progression-free survival (PFS), time to progression (TTP), and overall survival (OS) as defined by RECIST 1.1.

Objective response rate (ORR): the proportion of cases with a confirmed best response of CR or PR. The objective response of the tumor is assessed using the Response Evaluation Criteria in Solid Tumors (RECIST version 1.1 criteria). Patients must have measurable tumor lesions at baseline. The efficacy evaluation criteria are classified as complete response (CR), partial response (PR), stable disease (SD), and progressive disease (PD) according to the RECIST version 1.1 criteria.

Duration of response (DOR): DOR is defined as the time from the first assessment of objective response to the first assessment of PD or any-cause death before PD, reflecting the duration of ORR.

Disease control rate (DCR): the proportion of patients whose tumors have shrunk or stabilized and have been maintained for a certain period of time, including cases of CR, PR, and SD, also referred to as clinical benefit rate (CBR).

Progression-free survival (PFS): the time from the first administration to the occurrence of objective progression or all-cause death of the tumor (whichever occurs first).

Overall survival (OS): the time from the date of the first administration to the occurrence of death from any cause. For patients who are still alive at the time of analysis, the date of their last contact is used as the cutoff date.

During the study, it is recommended to use the same imaging examination technique for the same site of the same patient.

### BRIEF DESCRIPTION OF THE DRAWINGS

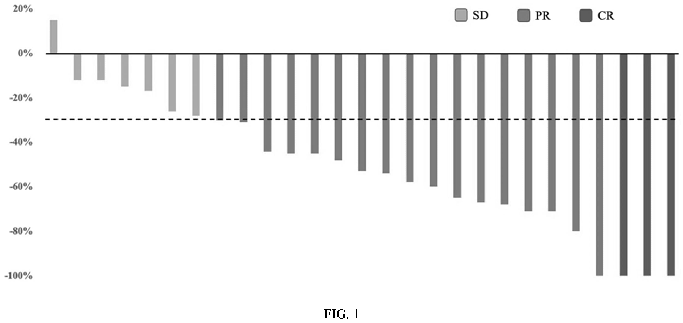

FIG. 1 shows the percentage of the best response of target lesions relative to the baseline in 27 subjects.

### DETAILED DESCRIPTION

The technical solution of the present disclosure will be further illustrated below through specific examples, which are not intended to limit the protection scope of the present disclosure. Some nonessential modifications and adjustments made by other people according to the concept of the present disclosure still fall within the protection scope of the present disclosure.

The materials, reagents, and the like used in the following examples can be commercially available, unless otherwise stated.

For the preparation method for antibody A, reference can be made to the patent application WO2020207432A1; the amino acid sequences of antibody A are shown in Table 1.

**Table 1. Amino acid sequences of antibody A**

| Name | SEQ ID NO | Amino acid sequence |
|---|---|---|
| HCDR1 | 1 | NYYMYW |
| HCDR2 | 2 | GINPSNGGTNFNEKF |
| HCDR3 | 3 | ARDYRLDMGFEF |
| LCDR1 | 4 | ASKGVSTSGYSYLH |
| LCDR2 | 5 | LASYLE |
| LCDR3 | 6 | YCQHAYDLPLT |
| VH | 7 | QVQLVQSGVEVKKPGASVKVSCKASGYTFTNYYMYWV RQAPGQGLEWMGGINPSNGGTNFNEKFKNRVTLTTDSST TTAYMELKSLQFDDTAVYYCAARDYRLDMGFEFWGQGT TVTVSS |
| VL | 8 | EIVLTQSPATLSLSPGERATLSCRASKGVSTSGYSYLHWY QQKPGQAPRLLIYLASYLESGVPARFSGSGSGTDFTLTISS LEPEDFAVYYCQHAYDLPLTFGGGTKVEIK |
| Heavy chain | 9 | QVQLVQSGVEVKKPGASVKVSCKASGYTFTNYYMYWV RQAPGQGLEWMGGINPSNGGTNFNEKFKNRVTLTTDSST TTAYMELKSLQFDDTAVYYCAARDYRLDMGFEFWGQGT TVTVSSASTKGPSVFPLAPCSRSTSESTAALGCLVKDYFPE PVTVSWNSGALTSGVHTFPAVLQSSGLYSLSSVVTVPSSSL GTKTYTCNVDHKPSNTKVDKRVESKYGPPCPPCPAPEFL GGPSVFLFPPKPKDTLMISRTPEVTCVVVDVSQEDPEVQF NWYVDGVEVHNAKTKPREEQFNSTYRVVSVLTVLHQD WLNGKEYKCKVSNKGLPSSIEKTISKAKGQPREPQVYTL PPSQEEMTKNQVSLTCLVKGFYPSDIAVEWESNGQPENN YKTTPPVLDSDGSFFLYSRLTVDKSRWQEGNVFSCSVMH EALHNHYTQKSLSLSLGK |
| Light chain | 10 | EIVLTQSPATLSLSPGERATLSCRASKGVSTSGYSYLHWY QQKPGQAPRLLIYLASYLESGVPARFSGSGSGTDFTLTISS LEPEDFAVYYCQHAYDLPLTFGGGTKVEIKRTVAAPSVFIF PPSDEQLKSGTASVVCLLNNFYPREAKVQWKVDNALQS GNSQESVTEQDSKDSTYSLSSTLTLSKADYEKHKVYACE VTHQGLSSPVTKSFNRGEC |

### Example 1: Phase II/III Study on Safety and Efficacy of Antibody A in Combination with Chemotherapy ± Bevacizumab for First-line Treatment of PD-L1-Positive (CPS ≥1) Persistent, Recurrent, or Metastatic Cervical Cancer

This study was a phase II/III seamless clinical trial. The phase II study was a single-arm, openlabel, and multicenter study on the safety and preliminary efficacy of antibody A in combination with chemotherapy ± bevacizumab, exploring the safety and preliminary efficacy of antibody A in combination with chemotherapy ± bevacizumab. If the safety of the combination regimen was controllable and the efficacy met expectations (i.e., the ORR being about 60% at 18 weeks (W)), the study proceeded to the phase III study. The phase III study was a randomized, double-blind, and multicenter clinical study on antibody A in combination with chemotherapy ± bevacizumab vs. chemotherapy ± bevacizumab for first-line treatment of PD-L1-positive (CPS ≥ 1) persistent, recurrent, or metastatic cervical cancer. Here, "± bevacizumab" indicated "with or without bevacizumab".

Compared to the first-line treatment with chemotherapy ± bevacizumab, it was expected in this study that the administration of antibody A in combination with chemotherapy ± bevacizumab would prolong the PFS and OS of the patient. Compared to the first-line treatment with chemotherapy ± bevacizumab, it was expected in this study that the administration of antibody A and chemotherapy ± bevacizumab would result in an improvement in at least one selected from the following: objective response rate (ORR), disease control rate (DCR), duration of response (DOR), time to progression (TTP), progression-free survival (PFS), and overall survival (OS).

### 1. Inclusion and exclusion criteria for patients:

### Inclusion criteria:

1. Aged ≥ 18 years and ≤ 70 years, female, voluntarily signed the informed consent form;
2. Histologically confirmed (the pathological report should be provided) persistent, recurrent, or metastatic (International Federation of Gynecology and Obstetrics [FIGO] stage IVB) cervical cancer, with pathological types including squamous cell carcinoma, adenocarcinoma, adenosquamous carcinoma, and clear cell carcinoma; not amenable to radical surgery and/or radical radiotherapy or chemoradiotherapy, and not received any systemic anti-tumor treatment for recurrent or metastatic cervical cancer;
3. Patients are required to meet the requirement of positive PD-L1 expression (CPS ≥ 1) as tested by the tumor specimen central laboratory. Patients should provide sufficient formalin-fixed paraffin-embedded (FFPE) specimens or sections (6 sections recommended, no less than 3 sections) and be willing to undergo a tumor tissue biopsy if necessary for PD-L1 testing. Archived tissues must be representative tumor specimens within three years or unstained serial sections of FFPE tumor tissues freshly resected within half a year, and the relevant pathological reports of the above specimens should also be provided. Both surgical resection and biopsy are acceptable methods for obtaining fresh tissue specimens; samples obtained via fine needle aspiration and liquid-based cytology test (TCT) (i.e., samples lacking complete tissue structures and only providing cell suspensions and/or cell smears) are not accepted; decalcified bone metastasis tumor tissue specimens are not accepted;
4. At least one measurable tumor lesion is present according to the Response Evaluation Criteria in Solid Tumors (RECIST 1.1); a lesion that has been previously treated with radiotherapy or other local regional therapies can only be considered as a non-target lesion, unless the lesion has clear progression or the specific tumor activity is confirmed via biopsy, and the lesion is measurable;
5. Expected survival ≥ 12 weeks as assessed by the investigator;
6. The U.S. Eastern Cooperative Oncology Group (ECOG) performance status scores 0 or 1;
7. Female patients with fertility must have a negative serum pregnancy test within 7 days prior to the first administration and be willing to take effective birth control/contraceptive methods to prevent pregnancy during the study until 6 months after the last administration of the study. Postmenopausal women must have amenorrhea for at least 12 months before they are considered infertile.

### Exclusion criteria:

1. Patients with other pathological types of cervical cancer, such as small-cell carcinoma and sarcoma;
2. Pregnant and lactating women;
3. Received radiotherapy within 14 days prior to the first administration. Except for palliative area radiotherapy for bone metastases at which pain cannot be effectively controlled by systemic treatment or local pain relief (the radiotherapy region < 5% of the bone marrow region); received chemotherapeutic drugs for increasing the sensitization to radiotherapy within 14 days prior to the first administration; patients who have received Chinese patent medicines or treatments having anti-tumor-related functions specified in the NMPA-approved drug instructions within 14 days prior to the first administration, or treatments with Chinese herbal medicines for anti-tumor purposes clearly documented in the medical records;
4. Received live/attenuated vaccines and mRNA vaccines within 4 weeks prior to screening or planned to receive live/attenuated vaccines and mRNA vaccines during the study;
5. Previously received any treatment targeting tumor immune mechanisms of action, such as treatment with an immune checkpoint inhibitor (e.g., an anti-PD-1 antibody, an anti-PD-L1 antibody, or an anti-CTLA-4 antibody) or treatment targeting immune co-stimulatory factors (e.g., antibodies directed against ICOS, CD40, CD137, GITR, or OX40 target).

### 2. Administration method:

The order of administration was as follows: 1) antibody A or placebo, 2) paclitaxel, 3) cisplatin or carboplatin, and 4) bevacizumab.
1) Antibody A or placebo: intravenous drip, with the total volume of the solution being 100 mL after preparation at a recommended dose of 300 mg, and the concentration range being controlled at 1-10 mg/mL. Dilution was performed with a 5% glucose solution. The prepared antibody A injection was administered intravenously, which was recommended to be administered by intravenous infusion for 30 minutes-1 hour, once every 3 weeks (21 days) on the first day of each cycle. The longest conventional administration duration in the study was about 2 years, with about 35 administration cycles.
2) Chemotherapy:
   Paclitaxel for injection (or paclitaxel liposome for injection): Administration was performed by intravenous infusion at 175 mg/m², once every 3 weeks (Q3W) on the first day of each cycle for a total of 6 cycles.
   Cisplatin: Administration was performed by intravenous infusion at 50 mg/m² once every 3 weeks (Q3W) immediately after the completion of paclitaxel infusion on the first day of each cycle; or administration was performed on the second day of each cycle with a combination of hydration therapy and diuresis for a total of 6 cycles. Patients may require a switch from cisplatin to carboplatin during the trial if required by clinical factors (e.g., impaired renal function).
   Carboplatin (intolerant to cisplatin): Administration was performed by intravenous infusion at AUC 5 mg/mL/min, once every 3 weeks (Q3W) on the first day of each cycle for a total of 6 cycles.
3) Bevacizumab: The recommended dose was 15 mg/kg; administration was performed once every 3 weeks (15 mg/kg/Q3W). Administration was performed by intravenous infusion, and the duration of the first intravenous infusion should be 90 minutes. If the first infusion was well tolerated, the duration of the second infusion could be shortened to 60 minutes. If the 60-minute infusion was also well tolerated by patients, all subsequent infusions could be completed within 30 minutes. It was recommended to continue the treatment with bevacizumab until the occurrence of progressive disease or intolerable toxicity. Within the first 6 cycles, administration was performed by infusion immediately after administration of cisplatin or carboplatin.

### 3. Endpoints of phase II study:

The phase II study was a study on the safety and preliminary efficacy of antibody A in combination with chemotherapy ± bevacizumab.

The primary endpoints were safety endpoints: vital signs and physical examinations, various adverse events (AEs), clinical laboratory tests, clinical auxiliary tests (e.g., electrocardiograms), and the like.

### Secondary endpoints:

- Pharmacokinetic parameters after single administration and multiple administrations (mainly including: Cₘₐₓ, Tₘₐₓ, T_{1/2}, CL, Vd, Ke, MRT, AUC_{(0-τ)}, and AUC_{(0-∞)} for single administration; C_{max, ss}, C_{avg, ss}, C_{min, ss}, AUC_{(0-τ)ss}, AUC_{(0-∞)ss}, T_{max, ss}, T_{1/2, ss}, CL, Vss, Ke, MRT, accumulation index (Rac), and fluctuation index (DF) for multiple administrations.
- Immunogenicity evaluation indexes: anti-drug antibody (ADA)/neutralizing antibody (NAb).
- Clinical efficacy index: preliminary efficacy evaluated according to the Response Evaluation Criteria in Solid Tumors (RECIST 1.1). The primary index was ORR prior to 18 weeks; other secondary indexes included DCR, DOR, TTP, PFS, OS, and the like.
- Biomarker: the PD-L1 expression level in tumor tissues.

### 4. Endpoints of phase III study:

The phase III study was a confirmatory study on the safety and efficacy of antibody A in combination with chemotherapy ± bevacizumab for first-line treatment of PD-L1-positive (CPS ≥ 1) persistent, recurrent, or metastatic cervical cancer.

The primary endpoints were evaluated according to the Response Evaluation Criteria in Solid Tumors (RECIST 1.1), and the PFS and OS of PD-L1-positive (CPS ≥ 1) recurrent or metastatic cervical cancer in the first-line treatment with antibody A in combination with chemotherapy ± bevacizumab were compared with those of PD-L1-positive (CPS ≥ 1) recurrent or metastatic cervical cancer in the first-line treatment with chemotherapy ± bevacizumab.

### Secondary endpoints:

- Safety endpoints: vital signs and physical examinations, various adverse events (AEs), clinical laboratory tests, and clinical auxiliary tests (e.g., electrocardiograms).
- Efficacy evaluated according to the Response Evaluation Criteria in Solid Tumors (RECIST 1.1), including: ORR, DCR, DOR, TTP, and the like.
- Immunogenicity evaluation indexes: anti-drug antibody (ADA)/neutralizing antibody (NAb).
- Biomarker: the correlation of the PD-L1 expression level in tumor tissues with efficacy.

### 4. Results of phase II study

As of October 8, 2024, a total of 29 subjects were enrolled and all had received at least one combination therapy; most of the enrolled patients were patients who had experienced recurrence after previous treatment, with more than half of the patients having previously received multiple cycles of treatment with paclitaxel-based drugs (paclitaxel for injection) + platinum-based drugs (cisplatin and/or carboplatin) during the adjuvant/neoadjuvant phase or the radical chemoradiotherapy phase. The baseline characteristics of the enrolled subjects are shown in Table 2. Among them, 27 subjects completed at least one efficacy evaluation, and the DCR was 100%; of these subjects, 26 cases showed shrinkage of target lesions, and the shrinkage reached 30% or more in 20 cases. The preliminary ORR was 74% (20/27), and 3 cases achieved complete response (CR) (FIG. 1).

**Table 2. Baseline characteristics of enrolled subjects**

| **Baseline characteristics of patients** | | **Antibody A in combination with chemotherapy with or without bevacizumab** |
|---|---|---|
| | | **N=29** |
| Median age (range), years | | 53 (32-69) |
| ECOG score, n (%) | | |
| | 0 | 9 (31) |
| | 1 | 20 (69) |
| FIGO stage at initial diagnosis, n (%) | | |
| | I | 3 (13.3) |
| | II | 4 (13.8) |
| | III | 14 (49.3) |
| | IVA | 1 (3.4) |
| | IVB | 7 (24.1) |
| Disease state at enrollment, n (%) | | |
| Metastatic | | 7 (24.1) |
| Recurrence or persistence with distant metastasis | | 18 (62.1) |
| Recurrence or persistence without distant metastasis | | 4 (13.8) |
| Histopathological type, n (%) | | |
| | Squamous cell carcinoma | 24 (82.8) |
| | Adenocarcinoma | 4 (13.8) |
| | Adenosquamous carcinoma | 1 (3.4) |
| PD-L1 expression level, n (%) | | |
| | 1 to 10 | 14 (49.3) |
| | ≥ 10 | 15 (50.7) |
| Previously received neoadjuvant/adjuvant chemotherapy or chemoradiotherapy with paclitaxel + platinum-based regimen, n (%) | | |
| | Yes | 17 (58.6) |
| | No | 12 (41.4) |
| Whether bevacizumab was used after enrollment, n (%) | | |
| | Yes | 23 (79.3) |
| | No | 6 (20.7) |

Among the 27 subjects, 4 subjects withdrew from the study due to progressive disease PD, 6 subjects voluntarily withdrew, and 1 died (due to gastrointestinal bleeding). The remaining 16 subjects were still in the study and all had been enrolled for more than 18 weeks, among whom 9 had been enrolled for more than 27 weeks and 3 had been enrolled for more than 36 weeks.

The results are shown in Table 3. Among the antibody A in combination with chemotherapy and bevacizumab trial groups, 2 cases achieved complete response, 12 cases achieved partial response, 3 cases achieved stable disease, 1 case had progressive disease, 2 subjects voluntarily withdrew, and 1 patient died; among the antibody A in combination with chemotherapy trial groups, 1 case achieved complete response, 3 cases achieved partial response, 1 case achieved stable disease, and 1 case had progressive disease. All of these groups exhibited excellent preliminary efficacy. The ECOG score, disease state, histopathological type, PD-L1 expression level, and FIGO stage at initial diagnosis of the patients at enrollment had no effects on the therapeutic effect of the combination regimen.

**The combination therapy was safe and tolerable**. The most common adverse events were anemia (82.6%), leukopenia (55.2%), alopecia (51.7%), thrombocytopenia (48.3%), neutropenia (44.8%), and the like. Grade ≥ 3 TRAEs were 62.1%. The preliminary data showed that the overall AEs and the proportion of grade ≥ 3 TRAEs were similar to those in previous similar studies, and no new safety signals were found. In addition, in terms of irAEs (immune-related adverse events), the current incidence rate and specific events were also similar to those in similar studies. Currently, only 1 case of grade 3 irAE, which was a grade 3 oral ulcer, occurred, and the patient continued to receive treatment after improvement.

The above results show that regardless of whether the same chemotherapy regimen has been previously used as adjuvant/neoadjuvant chemotherapy or radical chemoradiotherapy, the combination regimens of antibody A in combination with chemotherapy and antibody A in combination with chemotherapy and bevacizumab can achieve excellent preliminary efficacy and have acceptable safety and tolerability.

**Table 3. Specific efficacy data for 27 subjects**

| **Subject** | **Previously received a TC-like regimen as adjuvant/neoadjuvant chemotherapy or chemoradiotherapy** | **Whether bevacizumab was used** | **First efficacy evaluation 9W** | **Second efficacy evaluation 18W** | **Third efficacy evaluation 27W** | **Fourth efficacy evaluation 36W** |
|---|---|---|---|---|---|---|
| 1 | No | Yes | PR | PR | PR | |
| 2 | Yes | Yes | SD | PD | | |
| 3 | No | Yes | PR | CR | | |
| 4 | Yes | Yes | CR | CR | Voluntary withdrawal | |
| 5 | Yes | Yes | PR | PR | PR | |
| 6 | Yes | Yes | PR | PR | | |
| 7 | Yes | Yes | SD | SD | SD | SD |
| 8 | Yes | No | SD | PD | | |
| 9 | No | Yes | PR | Patient died | | |
| 10 | Yes | No | SD | SD | PR | |
| 11 | Yes | No | PR | CR | | |
| 12 | Yes | No | PR | PR | PD | |
| 13 | No | Yes | SD | SD | Voluntary withdrawal | |
| 14 | No | No | PR | PR | PR | PR |
| 15 | No | Yes | PR | PR | Voluntary withdrawal | |
| 16 | Yes | Yes | PR | PR | PD | |
| 17 | No | Yes | PR | PR | PR | |
| 18 | No | Yes | PR | PR | | |
| 19 | No | No | PR | PR | PR | PR |
| 20 | Yes | Yes | PR | PR | Voluntary withdrawal | |
| 21 | No | Yes | PR | PR | | |
| 22 | Yes | Yes | PR | PR | PR | |
| 23 | No | Yes | PR | PR | PR | |
| 24 | Yes | Yes | SD | Voluntary withdrawal | | |
| 25 | Yes | Yes | SD | SD | | |
| 26 | Yes | Yes | PR | PR | | |
| 27 | Yes | Yes | SD | Voluntary withdrawal | | |

### Example 2: Phase II/III Study on Safety and Efficacy of Antibody A in Combination with Chemotherapy for First-Line Treatment of Advanced or Recurrent Mismatch Repair Protein-Deficient (dMMR) Endometrial Cancer

The phase II study was a single-arm safety and efficacy exploration study, exploring the safety and preliminary efficacy of antibody A in combination with chemotherapy. If the safety of the combination regimen was controllable and the efficacy met expectations, enrollment in phase II was stopped, and the study proceeded to the phase III study. The phase III study was a randomized, double-blind, and multicenter clinical study on antibody A in combination with chemotherapy vs. placebo + chemotherapy for first-line treatment of patients with advanced or recurrent dMMR endometrial cancer.

Compared to patients administered only a chemotherapeutic agent, it was expected in this study that the administration of antibody A in combination with a chemotherapeutic agent would prolong the progression-free survival (PFS) of the patient. Compared to patients administered only a chemotherapeutic agent, the administration of antibody A in combination with a chemotherapeutic agent was expected to result in an improvement in at least one selected from the following: progression-free survival (PFS), objective response rate (ORR), disease control rate (DCR), duration of response (DOR), time to progression (TTP), and overall survival (OS).

### 1. Inclusion and exclusion criteria for patients:

### Inclusion criteria:

1. Aged ≥ 18 years and ≤ 75 years, female, voluntarily signed the informed consent form;
2. Patients must have one of the following types of endometrial cancer: a) a newly diagnosed stage III disease (a lesion evaluated as measurable according to RECIST 1.1 after surgery or diagnostic biopsy), b) a newly diagnosed stage IV disease (with or without a lesion after surgery or diagnostic biopsy), and c) a recurrent disease (a lesion evaluated as measurable or non-measurable according to RECIST 1.1), with little likelihood of cure by surgery alone or in combination;
3. Not received first-line systemic anti-cancer treatment. For patients with recurrent diseases only, if they have received prior chemotherapy in an adjuvant setting (as part of early/adjuvant anti-cancer treatment) and the time interval between the last dose of chemotherapy and the day of subsequent recurrence is at least 6 months, the prior chemotherapy is allowed;
4. Patients are required to meet the requirement of mismatch repair protein deficiency (dMMR) as tested by the tumor specimen central laboratory. Patients should provide sufficient formalin-fixed paraffin-embedded (FFPE) specimens or sections (6 sections recommended, no less than 3 sections) and be willing to undergo a tumor tissue biopsy if necessary for MMR status testing. Archived tissues must be representative tumor specimens within three years or unstained serial sections of FFPE tumor tissues freshly resected within half a year, and the relevant pathological reports of the above specimens should also be provided. Both surgical resection and biopsy are acceptable methods for obtaining fresh tissue specimens; samples obtained via fine needle aspiration and liquid-based cytology test (TCT) (i.e., samples lacking complete tissue structures and only providing cell suspensions and/or cell smears) are not accepted; decalcified bone metastasis tumor tissue specimens are not accepted;
5. The U.S. Eastern Cooperative Oncology Group (ECOG) performance status scores 0 or 1.

### Exclusion criteria:

1. Patients with endometrial leiomyosarcoma or other high-grade sarcomas, or endometrial stromal sarcoma;
2. Pregnant and lactating women;
3. Received radical radiotherapy within 3 months prior to the first administration of the study drug. Note: Palliative radiotherapy of bone or palliative radiotherapy of superficial lesions is allowed, with the course of treatment referring to the local standard and having ended 14 days prior to the first administration. Radiotherapy covering more than 30% of the bone marrow region within 28 days prior to the first administration is not allowed; received chemotherapeutic drugs for increasing the sensitization to radiotherapy within 14 days prior to the first administration; patients who have received Chinese patent medicines or treatments having anti-tumor-related functions specified in the NMPA-approved drug instructions within 14 days prior to the first administration, or treatments with Chinese herbal medicines for anti-tumor purposes clearly documented in the medical records;
4. Participating in the treatment phase of another clinical study, or planned to start treatment in this study within 14 days from the end of drug treatment in the previous clinical study;
5. Received live/attenuated vaccines and mRNA vaccines within 4 weeks prior to screening or planned to receive live/attenuated vaccines and mRNA vaccines during the study;
6. Previously received any treatment targeting tumor immune mechanisms of action, such as treatment with an immune checkpoint inhibitor (e.g., an anti-PD-1 antibody, an anti-PD-L1 antibody, or an anti-CTLA-4 antibody) or treatment targeting immune co-stimulatory factors (e.g., antibodies directed against ICOS, CD40, CD137, GITR, or OX40 target).

### 2. Administration method:

The order of administration was as follows: 1) antibody A or placebo, 2) paclitaxel, and 3) carboplatin.
1) Antibody A or placebo: Administration was performed by intravenous infusion at 300 mg, once every 3 weeks (Q3W) on the first day of each cycle. The longest conventional administration duration in the study was about 2 years, with about 35 administration cycles. Dilution was performed with a 5% glucose solution. The prepared antibody A injection was administered intravenously, which was recommended to be administered by intravenous infusion for 30 minutes to 1 hour. The administration should not be performed by intravenous bolus injection or a single rapid intravenous injection.
2) Chemotherapy:
   Paclitaxel: Paclitaxel was administered by intravenous infusion at 175 mg/m², once every 3 weeks (Q3W) on the first day of each cycle for a total of 6 cycles. Prophylactic administration was performed on all participating patients prior to infusion to prevent severe hypersensitivity.
   Carboplatin: Carboplatin was administered by intravenous infusion at AUC 5 mg/mL/min, once every 3 weeks (Q3W) on the first day of each cycle for a total of 6 cycles.

### 3. Endpoints of phase II study:

The phase II study was a study on the safety and preliminary efficacy of antibody A in combination with chemotherapy.

The primary endpoints were safety endpoints: vital signs and physical examinations, various adverse events (AEs), clinical laboratory tests, clinical auxiliary tests (e.g., electrocardiograms), and the like.

### Secondary endpoints:

- Pharmacokinetic parameters after single administration and multiple administrations (mainly including: Cₘₐₓ, Tₘₐₓ, T_{1/2}, CL, Vd, Ke, MRT, AUC_{(0-τ)}, and AUC_{(0-∞)} for single administration; C_{max, ss}, C_{avg, ss}, C_{min, ss}, AUC_{(0-τ)ss}, AUC_{(0-∞)ss}, T_{max, ss}, T_{1/2, ss}, CL, Vss, Ke, MRT, accumulation index (Rac), and fluctuation index (DF) for multiple administrations
- Immunogenicity evaluation indexes: anti-drug antibody (ADA)/neutralizing antibody (NAb).
- Clinical efficacy index: preliminary efficacy evaluated according to the Response Evaluation Criteria in Solid Tumors (RECIST 1.1). The primary index was ORR prior to 18 weeks; other secondary indexes included DCR, DOR, TTP, PFS, OS, and the like.

### 4. Endpoints of phase III study:

The phase III study was a confirmatory study on the safety and efficacy of antibody A in combination with chemotherapy for first-line treatment of dMMR advanced or recurrent endometrial cancer.

The primary endpoints were evaluated according to the Response Evaluation Criteria in Solid Tumors (RECIST 1.1), and the PFS of advanced or recurrent dMMR endometrial cancer in the first-line treatment with antibody A in combination with chemotherapy was compared with that of advanced or recurrent dMMR endometrial cancer in the first-line treatment with chemotherapy.

### Secondary endpoints:

- Safety endpoints: vital signs and physical examinations, various adverse events (AEs), clinical laboratory tests, and clinical auxiliary tests (e.g., electrocardiograms).
- Efficacy evaluated according to the Response Evaluation Criteria in Solid Tumors (RECIST 1.1), including: OS, ORR, DCR, DOR, TTP, and the like.
- Immunogenicity evaluation indexes: anti-drug antibody (ADA)/neutralizing antibody (NAb).

## Claims

1. A method for treating a tumor, comprising: administering to a patient in need thereof an effective amount of an anti-PD-1 antibody and a chemotherapeutic agent, wherein
the anti-PD-1 antibody comprises HCDR1 set forth in SEQ ID NO: 1, HCDR2 set forth in SEQ ID NO: 2, HCDR3 set forth in SEQ ID NO: 3, LCDR1 set forth in SEQ ID NO: 4, LCDR2 set forth in SEQ ID NO: 5, and LCDR3 set forth in SEQ ID NO: 6;
the chemotherapeutic agent is a taxane, a platinum-based drug, or a combination thereof;
the tumor is cervical cancer or endometrial cancer.

2. The method according to claim 1, wherein a heavy chain variable region of the anti-PD-1 antibody comprises the sequence set forth in SEQ ID NO: 7, a sequence having at least 80% identity to the sequence set forth in SEQ ID NO: 7, or an amino acid sequence having one or more conservative amino acid substitutions as compared to the sequence set forth in SEQ ID NO: 7; and/or
a light chain variable region of the anti-PD-1 antibody comprises the sequence set forth in SEQ ID NO: 8, a sequence having at least 80% identity to the sequence set forth in SEQ ID NO: 8, or an amino acid sequence having one or more conservative amino acid substitutions as compared to the sequence set forth in SEQ ID NO: 8.

3. The method according to claim 1, wherein a heavy chain of the anti-PD-1 antibody comprises the sequence set forth in SEQ ID NO: 9, a sequence having at least 80% identity to the sequence set forth in SEQ ID NO: 9, or an amino acid sequence having one or more conservative amino acid substitutions as compared to the sequence set forth in SEQ ID NO: 9; and/or
a light chain of the anti-PD-1 antibody comprises the sequence set forth in SEQ ID NO: 10, a sequence having at least 80% identity to the sequence set forth in SEQ ID NO: 10, or an amino acid sequence having one or more conservative amino acid substitutions as compared to the sequence set forth in SEQ ID NO: 10.

4. The method according to any one of claims 1-3, wherein the chemotherapeutic agent is a combination of a taxane and a platinum-based drug.

5. The method according to claim 4, wherein the chemotherapeutic agent is selected from the following groups: paclitaxel and cisplatin, paclitaxel and carboplatin, paclitaxel liposome and cisplatin, and paclitaxel liposome and carboplatin.

6. The method according to any one of claims 1-5, wherein the tumor is cervical cancer.

7. The method according to claim 6, wherein the cervical cancer comprises squamous cell carcinoma, adenocarcinoma, adenosquamous carcinoma, and clear cell carcinoma.

8. The method according to claim 6 or 7, wherein the cervical cancer comprises metastatic cervical cancer, recurrent cervical cancer, and persistent cervical cancer.

9. The method according to any one of claims 6-8, wherein the cervical cancer is PD-L1-positive (CPS ≥ 1) cervical cancer.

10. The method according to claim 6, wherein the cervical cancer is PD-L1-positive (CPS ≥ 1) metastatic cervical cancer, recurrent cervical cancer, or persistent cervical cancer.

11. The method according to any one of claims 1-5, wherein the tumor is endometrial cancer.

12. The method according to claim 11, wherein the endometrial cancer is recurrent or metastatic endometrial cancer.

13. The method according to claim 11, wherein the endometrial cancer is dMMR recurrent or metastatic endometrial cancer.

14. The method according to any one of claims 1-13, further comprising administering bevacizumab to the patient.

15. The method according to any one of claims 1-13, further comprising administering bevacizumab to the patient at a dose of about 15 mg/kg once every 3 weeks.

16. The method according to any one of claims 1-15, wherein the anti-PD-1 antibody is administered at a dose of about 50-600 mg.

17. The method according to claim 16, wherein the anti-PD-1 antibody is administered at a dose of about 200-400 mg.

18. The method according to claim 16, wherein the anti-PD-1 antibody is administered at a dose of about 200-220 mg once every 2 weeks.

19. The method according to claim 16, wherein the anti-PD-1 antibody is administered at a dose of about 300 mg once every 3 weeks.

20. The method according to claim 16, wherein the anti-PD-1 antibody is administered at a dose of about 380-400 mg once every 4 weeks.

21. The method according to any one of claims 4-20, wherein the paclitaxel is administered at a dose of about 50-300 mg/m² based on body surface area.

22. The method according to claim 21, wherein the paclitaxel is administered at a dose of about 135-175 mg/m² based on body surface area.

23. The method according to claim 21, wherein the paclitaxel is administered at a dose of about 175 mg/m² based on body surface area once every 3 weeks.

24. The method according to any one of claims 4-23, wherein the cisplatin is administered at a dose of about 10-100 mg/m² based on body surface area.

25. The method according to claim 24, wherein the cisplatin is administered at a dose of about 50 mg/m² based on body surface area once every 3 weeks.

26. The method according to any one of claims 4-25, wherein the carboplatin is administered at a dose achieving an AUC of about 4-7 mg/mL/min.

27. The method according to claim 26, wherein the carboplatin is administered at a dose achieving an AUC of about 5 mg/mL/min once every 3 weeks.

28. A method for treating cervical cancer, comprising: (1) administering to a patient an anti-PD-1 antibody at a dose of about 300 mg once every 3 weeks, (2) administering paclitaxel or paclitaxel liposome once every 3 weeks, and (3) administering cisplatin or carboplatin once every 3 weeks, wherein
the paclitaxel is administered at a dose of about 175 mg/m² based on body surface area;
the cisplatin is administered at a dose of about 50 mg/m² based on body surface area;
the carboplatin is administered at a dose of AUC 5 mg/mL/min;
the anti-PD-1 antibody comprises HCDR1 set forth in SEQ ID NO: 1, HCDR2 set forth in SEQ ID NO: 2, HCDR3 set forth in SEQ ID NO: 3, LCDR1 set forth in SEQ ID NO: 4, LCDR2 set forth in SEQ ID NO: 5, and LCDR3 set forth in SEQ ID NO: 6.

29. A method for treating cervical cancer, comprising: (1) administering to a patient an anti-PD-1 antibody at a dose of about 300 mg once every 3 weeks, (2) administering paclitaxel or paclitaxel liposome once every 3 weeks, (3) administering cisplatin or carboplatin once every 3 weeks, and (4) administering bevacizumab at a dose of about 15 mg/kg once every 3 weeks, wherein
the paclitaxel is administered at a dose of about 175 mg/m² based on body surface area;
the cisplatin is administered at a dose of about 50 mg/m² based on body surface area;
the carboplatin is administered at a dose of AUC 5 mg/mL/min;
the anti-PD-1 antibody comprises HCDR1 set forth in SEQ ID NO: 1, HCDR2 set forth in SEQ ID NO: 2, HCDR3 set forth in SEQ ID NO: 3, LCDR1 set forth in SEQ ID NO: 4, LCDR2 set forth in SEQ ID NO: 5, and LCDR3 set forth in SEQ ID NO: 6.

30. A method for treating endometrial cancer, comprising: (1) administering to a patient an anti-PD-1 antibody at a dose of about 300 mg once every 3 weeks, (2) administering paclitaxel once every 3 weeks, and (3) administering carboplatin once every 3 weeks, wherein
the anti-PD-1 antibody comprises HCDR1 set forth in SEQ ID NO: 1, HCDR2 set forth in SEQ ID NO: 2, HCDR3 set forth in SEQ ID NO: 3, LCDR1 set forth in SEQ ID NO: 4, LCDR2 set forth in SEQ ID NO: 5, and LCDR3 set forth in SEQ ID NO: 6;
the paclitaxel is administered at a dose of about 175 mg/m² based on body surface area;
the carboplatin is administered at a dose of AUC 5 mg/mL/min.

31. The method according to any one of claims 28-30, wherein a heavy chain variable region of the anti-PD-1 antibody comprises the sequence set forth in SEQ ID NO: 7, a sequence having at least 80% identity to the sequence set forth in SEQ ID NO: 7, or an amino acid sequence having one or more conservative amino acid substitutions as compared to the sequence set forth in SEQ ID NO: 7; and/or
a light chain variable region of the anti-PD-1 antibody comprises the sequence set forth in SEQ ID NO: 8, a sequence having at least 80% identity to the sequence set forth in SEQ ID NO: 8, or an amino acid sequence having one or more conservative amino acid substitutions as compared to the sequence set forth in SEQ ID NO: 8.

32. The method according to any one of claims 28-30, wherein a heavy chain of the anti-PD-1 antibody comprises the sequence set forth in SEQ ID NO: 9, a sequence having at least 80% identity to the sequence set forth in SEQ ID NO: 9, or an amino acid sequence having one or more conservative amino acid substitutions as compared to the sequence set forth in SEQ ID NO: 9; and/or
a light chain of the anti-PD-1 antibody comprises the sequence set forth in SEQ ID NO: 10, a sequence having at least 80% identity to the sequence set forth in SEQ ID NO: 10, or an amino acid sequence having one or more conservative amino acid substitutions as compared to the sequence set forth in SEQ ID NO: 10.
